# EUROPÄISCHE PATENTSCHRIFT

(11) **EP 3 861 976 B1**
(45) Veröffentlichungstag und Bekanntmachung des Hinweises auf die Patenterteilung: **30.03.2022**
(21) Anmeldenummer: 20155585.1
(22) Anmeldetag: 05.02.2020
(51) Int. Cl.: A61K 6/20, A61K 6/61, A61K 6/71, A61K 6/887, C07C 69/604, C07C 69/618, C08F 222/10

(54) **DENTALMATERIALIEN AUF BASIS VON CYCLOPOLYMERISIERBAREN VERNETZERN**
DENTAL MATERIALS BASED ON CYCLOPOLYMERISABLE CROSS-LINKING AGENTS
MATÉRIAUX DENTAIRES À BASE D'AGENTS DE RÉTICULATION CYCLOPOLYMÉRISABLES

(43) Veröffentlichungstag der Anmeldung: 11.08.2021
(73) Patentinhaber: Ivoclar Vivadent AG, 9494 Schaan (LI); Technische Universität Wien, 1040 Wien (AT)
(72) Erfinder: Lamparth, Iris, 9472 Grabs (CH); Moszner, Norbert, 9495 Triesen (LI); Liska, Robert, 2123 Schleinbach (AT); Gorsche, Christian, 1140 Wien (AT); Catel, Yohann, 9475 Ras (Sevelen) (CH); Peer, Gernot, 1100 Wien (AT)
(74) Vertreter: Uexküll & Stolberg

(56) Entgegenhaltungen:
- WO-A1-2014/040729
- WO-A1-2018/109041
- US-A- 5 145 374
- US-A1- 2012 082 958
- US-A1- 2019 117 523

## Beschreibung

Die Erfindung betrifft radikalisch polymerisierbare Zusammensetzungen, die sich besonders als Dentalwerkstoffe, wie z.B. Zemente, Füllungskomposite, Verblendmaterialien und Werkstoffe zur Herstellung von Inlays, Onlays, Kronen und Brücken eignen. Die Zusammensetzungen enthalten einen cyclopolymerisierbaren Vernetzer der Formel I und zeichnen sich durch einen geringen Polymerisationsschrumpf aus.

Die Polymerisation z.B. von Vinylverbindungen oder (Meth)acrylaten führt bekanntlich zu einer deutlichen Volumenkontraktion, da bei der Bildung der Polymerketten pro Kettenwachstumsschritt bei jedem Monomermolekül jeweils eine Doppelbindung und eine Van-der-Waals-Bindung in zwei Einfachbindungen überführt werden, d.h. die Monomerbausteine in der Polymerkette im Vergleich zur Monomerphase näher zusammenrücken. Dabei kann der Polymerisationsschrumpf zu nachteiligen Schrumpfungsspannungen, zur Spaltbildung oder verminderten Substrathaftung sowie zur Beeinträchtigung der Dimensionsstabilität von Formkörpern führen. Die Dichteänderung während der Polymerisation ist maßgeblich von der Molmasse und dem Molvolumen der Monomeren abhängig. Höhermolekulare Monomere zeigen im Vergleich zu Monomeren mit geringer Molmasse eine geringere Volumenkontraktion.

Im Dentalbereich finden verbreitet als relativ schrumpfungsarme Monomere die höhermolekularen Dimethacrylate Bis-GMA (Molmasse = 512,6 g/mol) und UDMA (Molmasse = 470,6 g/mol) Anwendung, die einen Polymerisationsschrumpf ΔV_{P} von 6,0 (Bis-GMA) bzw. 6,1 Vol.-% zeigen. Da diese Monomeren aber eine sehr hohe Viskosität aufweisen (Bis-GMA: η = 800-1000 Pa·s; UDMA: η = 10 Pa·s), werden sie meist in Mischung mit niedrigviskosen Dimethacrylaten mit geringerer Molmasse eingesetzt, die als Verdünner dienen. Monomere mit geringer Molmasse, wie z.B. Triethylenglycoldimethacrylat (Molmasse = 286,3 g/mol), haben aber einen erhöhten Polymerisationsschrumpf (ΔVₚ = 14,5 Vol.-%), was sich nachteilig auf den Polymerisationsschrumpf des Werkstoffs auswirkt.

Zur Herstellung von schrumpfungsarmen Polymerisaten wurden verschiedene Wege verfolgt, wie z.B. die Ringöffnungspolymerisation von cyclischen Monomeren oder der Einsatz von Thiol-En-Harzen.

Im Vergleich zu linearen Monomeren ist die Volumenkontraktion bei der Ringöffnungspolymerisation von cyclischen Monomeren deutlich geringer, weil hierbei pro Wachstumsschritt jeweils eine kovalente Bindung geöffnet und eine kovalente Bindung gebildet wird. Dementsprechend haben cyclische Monomere als schrumpfungsarme Matrixsysteme großes Interesse gefunden. Jedoch sind cyclische Monomere wie Spiroorthocarbonate, cyclische Ketenacetale oder Vinylcyclopropane im Vergleich zu Methacrylaten deutlich teurer und haben zum Teil nur eine beschränkte Lagerstabilität.

Die vernetzende Thiol-En-Polyaddition zeichnet sich im Vergleich zur radikalischen Polymerisation von multifunktionellen Methacrylaten durch einen nahezu vollständigen Doppelbindungsumsatz und einen deutlich geringeren Polymerisationsschrumpf aus. So beträgt die Volumenkontraktion pro polymerisierter (Meth)acrylat-Doppelbindung ca. 22-23 cm³/mol, während bei der Thiol-En-Reaktion die Volumenkontraktion nur bei 12-15 cm³ pro Mol umgesetzter Doppelbindung liegt. Außerdem verläuft die vernetzende Thiol-En-Polyaddition nach einem Stufenwachstumsmechanismus und hat deshalb eine im Vergleich zur Dimethacrylatpolymerisation signifikant verlängerte Prägelphase, was zusätzlich zur Verringerung der Polymerisationskontraktionsspannung führt. Leider ist der Einsatz von Thiol-En-Harzen durch den sehr unangenehmen Geruch der Thiole, die inhärente Flexibilität der Thiol-En-Polymerisate und durch die begrenzte Lagerstabilität der Thiol-En-Harze beschränkt.

Im Zusammenhang mit der Verringerung des Polymerisationsschrumpfes haben auch cyclopolymerisierbare Monomere Erwähnung gefunden. Dabei handelt es sich um di- oder höherfunktionelle Monomere, bei deren Polymerisation aufgrund ihrer speziellen Monomerstruktur neben der intermolekularen Kettenwachstumsreaktion eine intramolekularen Reaktion unter Bildung von 5- oder 6-gliedrigen Ringen stattfindet (vgl. Übersicht: D. Pasini, D Takeuchi, Chem. Rev. 118 (2018) 8993-9057). Die Cyclopolymerisation wurde 1957 von Butler erstmals für quarternäre Diallylammoniumsalze beschrieben. Weitere Beispiele für einfache cyclopolymerisierbare Monomere sind Acryl- und Methacrylsäureanhydrid, Methylallylmaleat und -fumarat, Diallylphthalat und Diethylenglycolbisallylcarbonat.

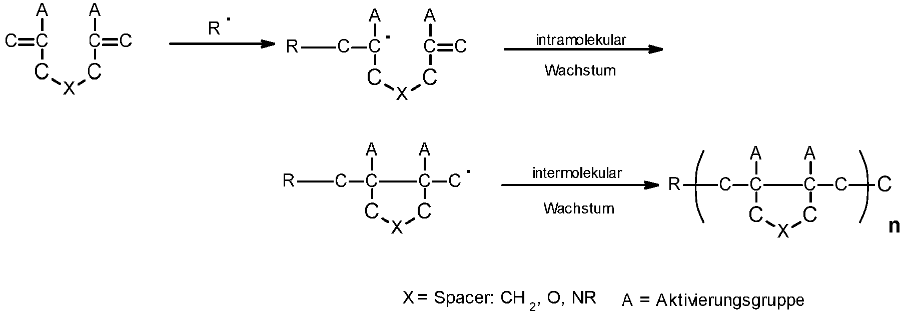

Die US 5,145,374 und die US 5,380,901 offenbaren dentale Adhäsive und Komposite, die cyclopolymerisierbare Bisacrylate (I) bzw. Oligomere (II) enthalten. Die Materialien sollen einen geringen Polymerisationsschrumpf aufweisen.

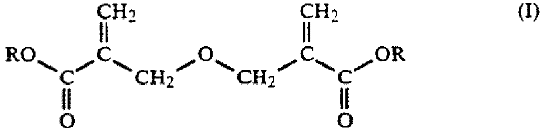

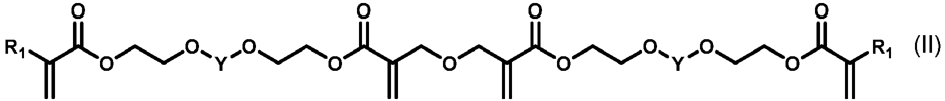

Die EP 3 335 688 A1 offenbart Dentalwerkstoffe, die cyclopolymerisierbare Monomere, wie z.B. 1,6-Dien-2-carbonsäure(ester)- und 1,5-Dien-2-carbonsäure(ester)-Monomere enthalten. Konkret werden u.a. α-Allyloxymethacrylsäurecyclohexylester und Methyl-α-allyloxymethylmethacrylat genannt. Die Werkstoffe sollen vor der Härtung eine für dentale Zwecke geeignete Fließfähigkeit und nach der Härtung eine hohe mechanische Festigkeit aufweisen, die auf die Ringbildung bei der Cyclopolymerisation zurückgeführt wird.

Die WO 2014/040729 A1 offenbart Dentalwerkstoffe, die N-allylsubstituierte (Meth)acrylamide enthalten, wie z.B. N,N,-Di(allylacrylamido)propan. Die N-allylsubstituierten (Meth)acrylamide sollen sich durch eine hohe Hydrolysestabilität, gute Copolymerisierbarkeit mit konventionellen (Meth)acrylaten, eine geringe Viskosität und ausgezeichnete Bioverträglichkeit auszeichnen.

Die WO 2018/109041 A1 offenbart Dentalwerkstoffe, die N-allylsubstituierte (Meth)acrylamide mit Phosphorsäureestergruppen enthalten, wie z.B. N-Acryl-8-allylaminooctylphosphorsäureester. Die (Meth)acrylamide sollen sich im Vergleich zu 10-Methyacryloyloxydecyldihydrogenphosphat durch eine hohe chemische Reinheit und eine hohe Polymerisationswärme auszeichnen. Außerdem sollen sie nach der Härtung vorteilhafte mechanische Eigenschaften ergeben.

Die US 2012/082958 A1 offenbart dentale Verbundwerkstoffe mit radikal polymerisierbaren Monomeren, die mindestens ein polyalizyklisches Strukturelement und ethylenisch ungesättigte Gruppen enthalten. Die dentalen Verbundwerkstoffe sollen sich durch einen geringen Polymerisationsschrumpf sowie eine hohe Abriebfestigkeit und Oberflächenhärte auszeichnen. Nachteilig an den bekannten cyclopolymerisierbaren Monomeren im Vergleich zu den üblicherweise zur Herstellung von Dentalwerkstoffen verwendeten (Meth)-acrylaten ist ihre deutliche geringere Reaktivität bei der radikalischen Polymerisation, was auf einen degradativen Kettenübertragungsmechanismus zurückzuführen ist. Außerdem sind ihre mechanischen Eigenschaften unbefriedigend.

Der Erfindung liegt die Aufgabe zugrunde, Dentalwerkstoffe zur Verfügung zu stellen, die sich durch einen geringen Polymerisationsschrumpf, gute mechanische Eigenschaften und eine hohe Reaktivität bei der radikalischen Polymerisation auszeichnen, insbesondere bei der Photopolymerisation.

Die Aufgabe wird erfindungsgemäß durch Dentalwerkstoffe gelöst, die mindestens eine Verbindung der Formel I enthalten: 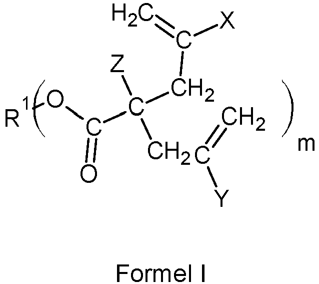 in der
- R¹: ein m-wertiger, linearer, verzweigter oder cyclischer aliphatischer C₁-C₃₀-Kohlenwasserstoffrest oder ein aromatischer C₆-C₃₀-Kohlenwasserstoffrest ist, wobei der aliphatische oder aromatische Kohlenwasserstoffrest unsubstituiert oder durch einen oder mehrere Substituenten substituiert sein kann, und wobei aliphatische Kohlenwasserstoffreste durch eine oder mehrere, vorzugsweise 1 bis 3, Urethangruppen, Estergruppen, Sauerstoffatome und/oder Schwefelatome unterbrochen sein können,
- X, Y, Z: unabhängig voneinander jeweils -COOR², -CON(R³R⁴), ein aromatischer C₆-C₁₀-Kohlenwasserstoffrest oder -CN sind, wobei
- R², R³, R⁴: jeweils unabhängig voneinander Wasserstoff, ein linearer, verzweigter oder cyclischer aliphatischer C₁-C₃₀-Kohlenwasserstoffrest, vorzugsweise C₁-C₁₀-Kohlenwasserstoffrest, oder ein aromatischer C₆-C₃₀-Kohlenwasserstoffrest, vorzugsweise C₁-C₁₀-Kohlenwasserstoffrest, sind, wobei der aliphatische oder aromatische Kohlenwasserstoffrest unsubstituiert oder durch einen oder mehreren Substituenten substituiert sein kann und wobei aliphatische Kohlenwasserstoffreste durch ein oder mehrere, vorzugsweise 1 bis 3, Sauerstoffatome, unterbrochen sein können, und
- m: 2 oder 3 ist.

Die bei den Resten R¹ bis R⁴ ggf. vorhandenen Substituenten sind vorzugsweise aus Chlor, Hydroxy und Methoxy ausgewählt, wobei die Reste ggf. vorzugsweise durch 1 bis 3 Substituenten substituiert. Die Reste R¹ bis R⁴ sind vorzugsweise nicht mit aciden Gruppen substituiert und besonders bevorzugt unsubstituiert.

Verbindungen, in denen m gleich 2 ist, lassen sich durch Formel la und Verbindungen, in denen m gleich 3 ist, durch Formel lb darstellen:

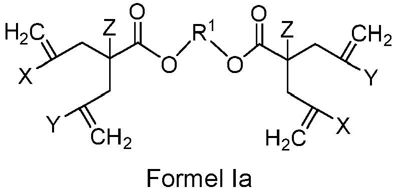

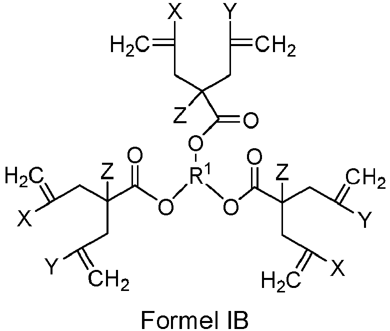

Die Formel I erstreckt sich nur auf solche Verbindungen, die mit der chemischen Valenzlehre vereinbar sind. Der Hinweis, dass ein Rest z.B. durch ein oder mehrere O-Atome unterbrochen ist, ist so zu verstehen, dass diese Atome oder Gruppen jeweils in die Kohlenstoffkette des Restes eingeschoben werden. Diese Atome oder Gruppen sind damit beidseitig durch C-Atome begrenzt und können nicht endständig sein. C₁-Reste können nicht unterbrochen, verzweigt oder cyclisch sein. Unter aromatischen Kohlenwasserstoffresten werden der üblichen Nomenklatur entsprechend auch solche Reste verstanden, die aromatische und nicht aromatische Gruppen enthalten. Ein bevorzugter aromatischer Rest ist beispielsweise der Diphenylpropanrest.

Die Gruppen X, Y und Z können gleich oder verschieden sein. Vorzugsweise haben X und Y dieselbe Bedeutung, besonders bevorzugt sind Verbindungen, in denen X, Y und Z gleich sind.

Vorzugsweise haben die Variablen die folgenden Bedeutungen:
- R¹: ein linearer, verzweigter oder cyclischer aliphatischer C₁-C₂₀-Kohlenwasserstoffrest, der durch eine oder mehrere, vorzugsweise 1 bis 3, Urethangruppen, Estergruppen und/oder Sauerstoffatome unterbrochen sein kann,
- X, Y, Z: unabhängig voneinander jeweils -COOR², ein aromatischer C₆-C₁₀-Kohlenwasserstoffrest oder -CN,
- R²: ein linearer, verzweigter oder cyclischer aliphatischer C₁-C₁₀-Kohlenwasserstoffrest, der durch ein oder mehrere, vorzugsweise 1 bis 3, Sauerstoffatome unterbrochen sein kann, und
- m: 2 oder 3.

Besonders bevorzugt sind Verbindung der Formel I, in der die Variablen die folgenden Bedeutungen haben:
- R¹: ein linearer, verzweigter oder cyclischer aliphatischer C₁-C₁₂-Kohlenwasserstoffrest, vorzugsweise ein gesättigter, verzweigter oder vorzugsweise linearer C₂- bis C₈-Kohlenwasserstoffrest, der durch ein oder mehrere, vorzugsweise 1 bis 3, Sauerstoffatome unterbrochen sein kann, und vorzugsweise nicht unterbrochen ist,
- X, Y, Z: unabhängig voneinander jeweils -COOR² oder Phenyl, vorzugsweise -COOR²,
- R²: ein linearer oder verzweigter C₁₋C₄-Kohlenwasserstoffrest, vorzugsweise Ethyl, und
- m: 2 oder 3, vorzugsweise 2.

Die für die einzelnen Variablen angegebenen bevorzugten, besonders bevorzugten und ganz besonders bevorzugten Definitionen können jeweils unabhängig voneinander ausgewählt werden. Verbindungen, in denen alle Variablen die bevorzugten, besonders bevorzugten und ganz besonders bevorzugten Definitionen aufweisen, sind naturgemäß erfindungsgemäß besonders geeignet.

Bei den Kohlenwasserstoffresten handelt es sich in allen Fällen vorzugsweise um gesättigte Kohlenwasserstoffreste. Dies gilt sowohl für die allgemeine Definition als auch für die bevorzugten und insbesondere auch für die besonders bevorzugten Verbindungen der Formel I.

Die cyclopolymerisierbaren Verbindungen der Formel I sind nicht bekannt und lassen sich in Anlehnung an ähnliche Verbindungen herstellen (vgl. Bourgeois, J.-P.; Echegoyen, L.; Fibbioli, M.; Pretsch, E.; Diederich, F., Angewandte Chemie International Edition 1998, 37 (15), 2118-2121 und Gregg, Z. R.; Griffiths, J. R.; Diver, S. T., Organometallics 2018, 37 (10), 1526-1533). Dabei wird im ersten Schritt ein multifunktioneller Alkohol unter basischen Bedingungen mit einem Säurechlorid verestert. Im zweiten Schritt wird nach Deprotonierung mit einer Vinylquelle auf Basis von 2-Chloromethylalkenen umgesetzt.
1. Schritt: 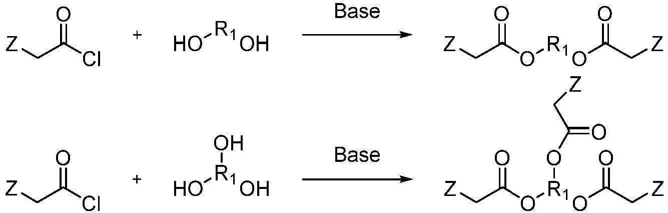
2. Schritt:

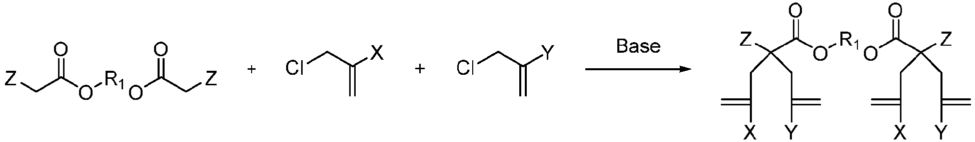

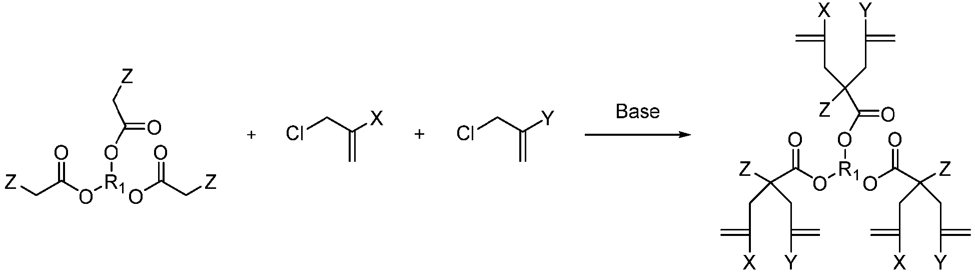

Ein konkretes Beispiel ist:
1) 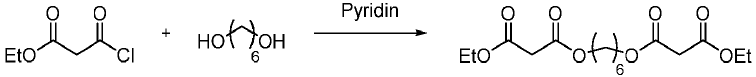
2)

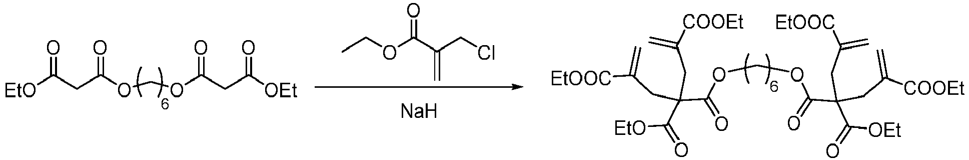

Bevorzugte erfindungsgemäße Verbindungen der Formel I sind: 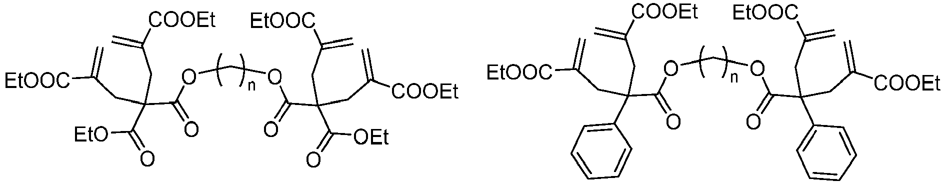 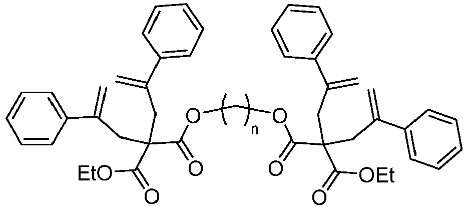 mit n = 1 bis 15

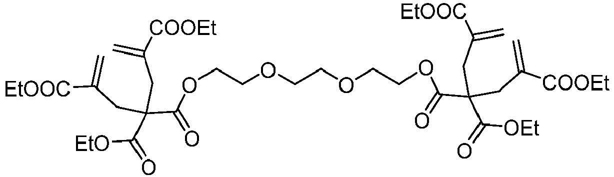

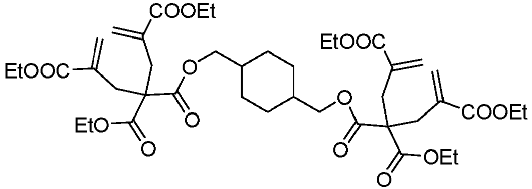

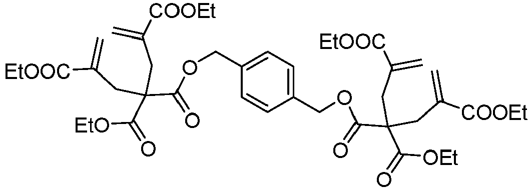

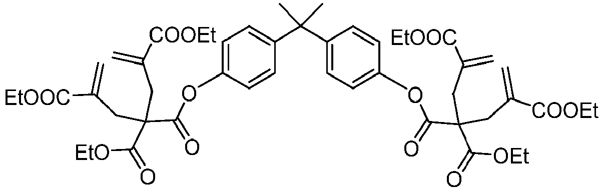

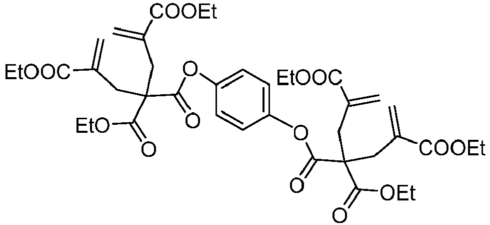

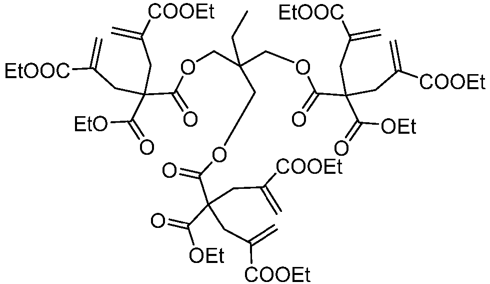

Besonders bevorzugte Verbindungen der Formel la sind die folgenden Substanzen:

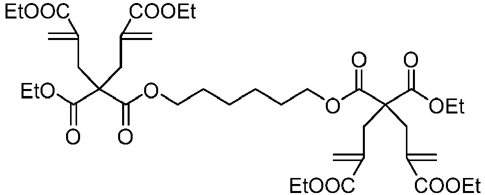

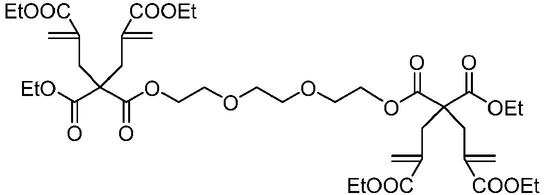

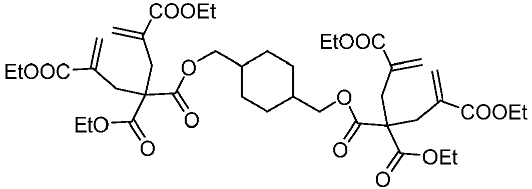

Eine bevorzugte Verbindung der Formel Ib ist:

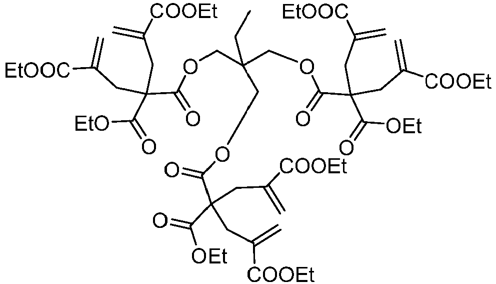

Die erfindungsgemäßen Verbindungen der Formel I enthalten vier oder sechs polymerisierbare Gruppen und haben vernetzende Eigenschaften. Sie zeichnen sich durch eine hohe Reaktivität der cyclopolymerisierbaren Gruppen bei der radikalischen Polymerisation aus und sind gut mit herkömmlichen Dentalmonomeren, insbesondere mit Di(meth)acrylaten, copolymerisierbar. Aufgrund ihrer hohen Reaktivität lassen sie sich im Gegensatz zu den bekannten, cyclopolymerisierbaren N,N-disubstituierten Methacrylamiden beispielsweise auch homopolymerisieren. Bei der Homo- und Copolymerisation der erfindungsgemäßen Verbindungen der Formel I werden Polymernetzwerke mit guten mechanischen Eigenschaften erhalten, die für dentale Anwendungen vorteilhaft sind.

Darüber hinaus zeichnen sich die erfindungsgemäßen Verbindungen der Formel I durch eine verminderte Polymerisationsschrumpfungsspannung und einen geringen Polymerisationsschrumpf infolge der Cyclopolymerisation aus. Sie ermöglichen es damit, diese Vorteile für dentale Anwendungen zu nutzen, ohne andere, für dentale Zwecke ebenfalls wesentliche Eigenschaften, wie insbesondere die mechanischen Eigenschaften, zu beeinträchtigen.

Die Verbindungen der Formel I eignen sich besonders zur Herstellung von Dentalwerkstoffen, beispielsweise zur Herstellung von Beschichtungs- oder Verblendmaterialien, dentalen Zementen und insbesondere von Füllungskompositen. Sie eignen sich weiterhin zur Herstellung von Werkstoffen zur Herstellung oder Reparatur von dentalen Prothesen, Inlays, Onlays, Kronen oder Brücken. Sie eignen sich aber auch zur Herstellung von radikalisch polymerisierbaren Werkstoffen und Duromeren für andere Zwecke. Die erfindungsgemäßen (Dental-)Werkstoffe enthalten vorzugweise 0,5 bis 70 Gew.-%, besonders bevorzugt 1 bis 60 Gew.-% und ganz besonders bevorzugt 3 bis 50 Gew.-% mindestens einer Verbindung der Formel I, bezogen auf die Gesamtmasse des Werkstoffs.

Die erfindungsgemäßen Verbindungen der Formel I werden vorzugsweise in Kombination mit einem oder mehreren polymerisationsfähigen, mono- oder multifunktionellen Monomeren (Comonomere) eingesetzt. Unter monofunktionellen Monomeren werden Verbindungen mit einer, und unter multifunktionellen Monomeren Verbindungen mit zwei oder mehr, vorzugsweise 2 bis 4 radikalisch polymerisierbaren Gruppen verstanden. Bevorzugte Comonomere sind radikalisch polymerisierbare Monomere.

Erfindungsgemäß sind Dentalwerkstoffe bevorzugt, die als radikalisch polymerisierbares Comonomer mindestens ein mono- oder multifunktionelles (Meth)acrylat enthalten. Materialien, die intraoral gehärtet werden sollen, enthalten als radikalisch polymerisierbares Monomer vorzugsweise mono- und/oder multifunktionelle Methacrylate. Methacrylate sind als Comonomere daher besonders bevorzugt. Es wurde überraschenderweise gefunden, dass die erfindungsgemäßen Verbindungen der Formel I mit herkömmlichen (Meth)acrylaten gut copolymerisierbar sind.

Bevorzugte multifunktionelle Methacrylate sind Dimethacrylate, insbesondere Bisphenol-A-dimethacrylat, 2,2-Bis[4-(2-hydroxy-3-methacryloyloxypropyl)phenyl]propan (Bis-GMA; ein Additionsprodukt aus Methacrylsäure und Bisphenol-A-diglycidylether), ethoxy- oder propoxyliertes Bisphenol-A-dimethacrylat, wie z.B. 2-[4-(2-Methacryloyloxyethoxyethoxy)phenyl]-2-[4-(2-methacryloyloxyethoxy)phenyl]propan) (SR-348c; enthält 3 Ethoxygruppen), 2,2-Bis[4-(2-methacryloyloxypropoxy)-phenyl]propan, 1,6-Bis-[2-methacryloyloxy-ethoxycarbonylamino]-2,2,4-trimethyl-hexan (UDMA; ein Additionsprodukt aus 2-Hydroxyethylmethacrylat und 2,2,4-Tri-methylhexamethylen-1,6-diisocyanat), Di-, Tri-und Tetraethylenglycoldimethacrylat, Trimethylolpropantrimethacrylat, Pentaerythrittetramethacrylat, Glycerindi- und Glycerintrimethacrylat, 1,4-Butandioldimethacrylat, 1,10-Decandioldimethacrylat (D₃MA), 1,12-Dodecandioldimethacrylat, Bis(methacryloyloxymethyl)tricyclo-[5.2.1.0^{2,6}]decan (DCP) und Mischungen davon. Besonders bevorzugte multifunktionelle Methacrylate sind Bis-GMA, SR-348c, UDMA, D₃MA, DCP, Triethylenglycoldimethacrylat und Mischungen davon.

Multifunktionelle Monomere, insbesondere Methacrylate, werden vorzugsweise in einer Menge von insgesamt maximal 70 Gew.-%, besonders bevorzugt 1,5 bis 60 Gew.-% und ganz besonders bevorzugt 2 bis 50 Gew.-% eingesetzt, bezogen auf die Gesamtmasse des Werkstoffs.

Erfindungsgemäß bevorzugte monofunktionelle Monomere sind Monomethacrylate. Besonders bevorzugte Monomethacrylate sind Benzyl-, Tetrahydrofurfuryl-, Isobornylmethacrylat, p-Cumyl-phenoxyethylenglycolmethacrylat (CMP-1E), 2-(2-Biphenyl-oxy)-ethylmethacrylat und Mischungen davon.

Monofunktionelle Monomere, insbesondere Methacrylate, werden vorzugsweise in einer Menge von insgesamt maximal 10 Gew.-%, besonders bevorzugt 0 bis 10 Gew.-% und ganz besonders bevorzugt 0 bis 5 Gew.-% eingesetzt, bezogen auf die Gesamtmasse des Werkstoffs.

Weiterhin sind als Comonomere auch mono- und multifunktionelle Acrylate geeignet. Bevorzugte multifunktionelle Acrylate sind Ethylenglycoldiacrylat, Hexandioldiacrylat, Tripropylenglycoldiacrylat, ethoxyliertes Bisphenol-A-diacrylat, Polyethylenglycol-200-diacrylat, Trimethylolpropantriacrylat, Pentaerythrittetraacrylat und Mischungen davon.

Mono- und multifunktionelle Acrylate, werden vorzugsweise in einer Menge von insgesamt maximal 50 Gew.-%, vorzugsweise 0 bis 50 Gew.-% und besonders bevorzugt 0 bis 30 Gew.-% eingesetzt, bezogen auf die Gesamtmasse des Dentalwerkstoffs.

Die Gesamtmenge an Comonomeren beträgt vorzugsweise maximal 70 Gew.-%, besonders bevorzugt 1,5 bis 60 Gew.-% und ganz besonders bevorzugt 2 bis 50 Gew.-%, bezogen auf die Gesamtmasse des Werkstoffs.

Die erfindungsgemäßen Zusammensetzungen enthalten vorzugsweise einen Initiator für die radikalische Polymerisation, z.B. für die Polymerisation durch UV-Licht, durch sichtbares Licht, einen thermischen Initiator und/oder einen Redoxinitiator. Photoinitiatoren sind besonders bevorzugt, ganz besonders bevorzugt sind Photoinitiatoren, die durch sichtbares Licht aktiviert werden.

Als UV-Photoinitiatoren sind Norrish-Typ-I-Initiatoren, wie z.B. Benzildimethylketal, Benzoinether, Hydroxyphenylketone, Dialkoxyacetophenone, Benzoylcyclohexanol, Trimethylbenzoylphosphinoxid und Morpholinophenylaminoketone bevorzugt. Bevorzugte Norrish-Typ-II-UV-Initiatoren sind Mischungen von z.B. Benzophenon oder Thioxanthon-Derivaten mit H-Donatoren wie Alkoholen, oder Thiolen bzw. Elektronendonatoren wie Aminen.

Bevorzugte bimolekulare Photoinitiatoren für den sichtbaren Bereich sind α-Diketone und dessen Derivate, wie 9,10-Phenanthrenchinon, 1-Phenyl-propan-1,2-dion, Diacetyl oder 4,4-Dichlorbenzil, sowie Mischungen davon. Besonders bevorzugt sind Campherchinon (CC) und 2,2-Dimethoxy-2-phenyl-acetophenon, und ganz besonders bevorzugt sind α-Diketone in Kombination mit Aminen als Reduktionsmittel, wie z.B. 4-(Dimethylamino)-benzoesäureethylester (EDMAB), N,N-Dimethylaminoethylmethacrylat, N,N-Dimethyl-sym.-xylidin oder Triethanolamin. Bevorzugte Norrish-Typ-I-Photoinitiatoren für den sichtbaren Bereich sind Bisacylphosphinoxide. Besonders bevorzugt sind Monoacyltrialkylgermanium-, Diacyldialkylgermanium- und Tetraacylgermanium-Verbindungen, wie z.B. Benzoyltrimethylgerman, Dibenzoyldiethylgerman, Bis(4-methoxybenzoyl)diethylgerman (Ivocerin^{®}), Tetrabenzoylgerman oder Tetrakis(o-methylbenzoyl)german.

Außerdem lassen sich vorteilhaft auch Mischungen der verschiedenen Photoinitiatoren einsetzen, wie z.B. Bis(4-methoxybenzoyl)diethylgerman bzw. Tetrakis(o-methyl-benzoyl)german in Kombination mit Campherchinon und 4-Dimethylaminobenzoesäureethylester.

Bevorzugte thermische Initiatoren sind Azoverbindungen, wie z.B. 2,2'-Azobis(iso-butyronitril) (AIBN), Azobis-(4-cyanovaleriansäure) oder Peroxide, wie z.B. Dibenzoylperoxid, Dilauroylperoxid, *tert*-Butylperoctoat, *tert*-Butylperbenzoat oder Di-(tert-butyl)-peroxid. Zur Beschleunigung der Initiierung mittels Peroxiden lassen sich auch Kombinationen von Peroxiden mit aromatischen Aminen einsetzen. Bevorzugte Kombinationen sind Kombinationen von Dibenzoylperoxid mit einem Amin, vorzugsweise einem N,N-dialkylsubstituierten aromatischen Amin, das in p-Stellung substituiert ist, wie N,N-Dimethyl-p-toluidin, N,N-Dihydroxyethyl-p-toluidin, p-Dimethylam inobenzoesäureethylester.

Für eine duale Aushärtung eignen sich Kombinationen eines Photoinitiators mit einem thermischen oder vorzugsweise einem Redoxinitiator. Bevorzugte Redoxinitiatoren für die duale Härtung sind Systeme, die ein Peroxid und ein Reduktionsmittel, wie z.B. Ascorbinsäure, ein Barbiturat oder eine Sulfinsäure, oder ein Hydroperoxid in Kombination mit einem Reduktionsmittel und ggf. katalytischen Mengen an Metallionen enthalten, wie z.B. eine Mischung von Cumolhydroperoxid, einem Thioharnstoffderivat und Kupfer(II)-acetylacetonat.

Die erfindungsgemäßen Zusammensetzungen können vorteilhaft außerdem einen oder mehrere organische oder vorzugsweise anorganische Füllstoffe enthalten. Bevorzugt sind faserförmige und insbesondere partikuläre Füllstoffe. Füllstoffhaltige Zusammensetzungen eignen sich besonders als dentale Befestigungszemente oder Füllungskomposite.

Als faserförmige Füllstoffe sind Glasfasern, Kohlenstofffasern, Keramik- und Aramidfasern sowie Nanofasern oder Whiskers bevorzugt. Faserförmige Füllstoffe eignen sich besonders zur Herstellung von Verbundmaterialien. Unter Nanofasern werden Fasern mit einer Länge von weniger als 100 nm verstanden und unter Whiskers nadelförmige Einkristalle, vorzugsweise aus Aluminiumoxid oder Siliciumcarbid. Whiskers haben typischerweise einen Durchmesser von wenigen µm und einer Länge bis zu 1 mm.

Bevorzugte partikuläre Füllstoffe sind Oxide, wie SiO₂, ZrO₂ und TiO₂ oder Mischoxide aus SiO₂, ZrO₂, ZnO und/oder TiO₂, nanopartikuläre oder mikrofeine Füllstoffe, wie pyrogene Kieselsäure oder Fällungskieselsäure, Glaspulver, wie Quarz-, Glaskeramik-, Borosilikat- oder röntgenopake Glaspulver, vorzugsweise Barium- oder Strontiumaluminiumsilikatgläser, und röntgenopake Füllstoffe, wie Ytterbiumtrifluorid, Tantal(V)-oxid, Bariumsulfat oder Mischoxide von SiO₂ mit Ytterbium(III)-oxid oder Tantal(V)-oxid.

Bevorzugt weisen die Oxide eine Partikelgröße von 0,010 bis 15 µm, die nanopartikulären oder mikrofeinen Füllstoffe eine Partikelgröße von 10 bis 300 nm, die Glaspulver eine Partikelgröße von 0,01 bis 15 µm, vorzugweise von 0,2 bis 1,5 µm, und die röntgenopaken Füllstoffe eine Partikelgröße von 0,2 bis 5 µm auf.

Besonders bevorzugte Füllstoffe sind Mischoxide aus SiO₂ und ZrO₂, mit einer Partikelgröße von 10 bis 300 nm, Glaspulver mit einer Partikelgröße von 0,2 bis 1,5 µm, insbesondere röntgenopake Glaspulver von z.B. Barium- oder Strontiumaluminiumsilikatgläsern, und röntgenopake Füllstoffe mit einer Partikelgröße von 0,2 bis 5 µm, insbesondere Ytterbiumtrifluorid und/oder Mischoxide von SiO₂ mit Ytterbium(III)-oxid.

Wenn nicht anders angegeben, handelt es sich bei allen Partikelgrößen um gewichtsmittlere Partikelgrößen (D50-Werte), wobei die Partikelgrößenbestimmung im Bereich von 0,1 µm bis 1000 µm vorzugsweise mittels statischer Lichtstreuung erfolgt, beispielsweise mit einem statischen Laserstreuungs-Partikelgrößen-Analysator LA-960 (Horiba, Japan). Hierbei werden als Lichtquellen eine Laser-Diode mit einer Wellenlänge von 655 nm und eine LED mit einer Wellenlänge von 405 nm verwendet. Der Einsatz von zwei Lichtquellen mit unterschiedlichen Wellenlängen ermöglicht die Vermessung der gesamten Partikelgrößenverteilung einer Probe in nur einem Messungsdurchgang, wobei die Messung als Nassmessung durchgeführt wird. Hierzu wird eine 0,1 bis 0,5%ige wässrige Dispersion des Füllstoffs hergestellt und deren Streulicht in einer Durchflusszelle gemessen. Die Streulichtanalyse zur Berechnung von Partikelgröße und Partikelgrößenverteilung erfolgt gemäß der Mie-Theorie nach DIN/ISO 13320. Die Messung der Partikelgröße im Bereich von 5 nm bis 0,1 µm erfolgt vorzugsweise durch Dynamische Lichtstreuung (DLS) von wässrigen Partikeldispersionen, vorzugsweise mit einem He-Ne-Laser mit einer Wellenlänge von 633 nm, bei einem Streuwinkel von 90° und bei 25°C, z.B. mit einem Malvern Zetasizer Nano ZS (Malvern Instruments, Malvern UK).

Partikelgrößen kleiner als 0,1 µm können auch anhand von REM- oder TEM-Aufnahmen bestimmt werden. Die Transmissionselektronenmikroskopie (TEM) wird vorzugsweise mit einem Philips CM30 TEM bei einer Beschleunigungsspannung von 300 kV durchgeführt. Für die Probenvorbereitung werden Tropfen der Partikeldispersion auf einem 50 Å dickem Kupfergitter (Maschenweite 300 Mesh) aufgebracht, das mit Kohlenstoff beschichtet ist, und im Anschluss das Lösungsmittel verdampft. Die Partikel werden ausgezählt und der arithmetische Mittelwert berechnet.

Die Füllstoffe werden nach ihrer Partikelgröße unterteilt in Makrofüller und Mikrofüller, wobei Füllstoffe mit einer mittleren Partikelgröße von 0,2 bis 15 µm als Makrofüller und Füllstoffe mit einer mittlere Partikelgröße von ca. 5 bis 100 nm als Mikrofüller bezeichnet werden. Makrofüller werden z.B. durch Mahlen z.B. von Quarz, röntgenopaken Gläsern, Borosilikaten oder von Keramik gewonnen und bestehen meist aus splitterförmigen Teilen. Mikrofüller wie Mischoxide können z.B. durch hydrolytische Co-Kon¬densation von Metallalkoxiden hergestellt werden.

Zur Verbesserung des Verbundes zwischen den Füllstoffpartikeln und der vernetzten Polymerisationsmatrix sind die Füllstoffe vorzugsweise oberflächenmodifiziert. SiO₂-basierende Füllstoffe sind vorzugsweise mit Methacrylat-funktionalisierten Silanen oberflächenmodifiziert, besonders bevorzugt mit 3-Methacryloyloxypropyltrimethoxysilan. Zur Oberflächenmodifizierung von nichtsilikatischen Füllstoffen, z.B. von ZrO₂ oder TiO₂ können auch funktionalisierte saure Phosphate, wie z.B. 10-Methacryloyloxydecyldihydrogenphosphat eingesetzt werden.

Außerdem können die erfindungsgemäßen Dentalwerkstoffe ein oder mehrere weitere Additive enthalten, vor allem Stabilisatoren, Farbmittel, mikrobiozide Wirkstoffe, fluoridionenabgebende Additive, Treibmittel, optische Aufheller, Weichmacher und/oder UV-Absorber.

Die erfindungsgemäßen Werkstoffe enthalten vorzugsweise:
a) 0,5 bis 70 Gew.-%, bevorzugt 1 bis 60 Gew.-% und besonders bevorzugt 3 bis 50 Gew.-% mindestens einer Verbindung der Formel I,
b) 0,01 bis 5 Gew.-%, bevorzugt 0,1 bis 3,0 Gew.-% und besonders bevorzugt 0,1 bis 1,0 Gew.-% mindestens eines Initiators für die radikalische Polymerisation, vorzugsweise eines Photoinitiators,
c) 1 bis 70 Gew.-%, bevorzugt 1,5 bis 60 Gew.-% und besonders bevorzugt 2 bis 50 Gew.-% mindestens eines radikalisch polymerisierbaren Comonomers,
d) 0 bis 85 Gew.-% mindestens eines Füllstoffs.

Alle Mengenangaben hierin beziehen sich auf die Gesamtmasse der Zusammensetzung, wenn nicht anders angegeben.

Der Füllungsgrad richtet sich nach dem gewünschten Anwendungszweck des Werkstoffs. Füllungskomposite haben vorzugsweise einen Füllstoffgehalt von 50 bis 85 Gew.-%, besonders bevorzugt 70 bis 80 Gew.-%, und dentale Zemente von 10 bis 70 Gew.-%, besonders bevorzugt 60 bis 70 Gew.-%.

Besonders bevorzugt sind solche Dentalwerkstoffe, die aus den genannten Komponenten bestehen, wobei die einzelnen Komponenten vorzugsweise jeweils aus den oben genannten bevorzugten und besonders bevorzugten Stoffen ausgewählt sind.

Die erfindungsgemäßen Zusammensetzungen eignen sich besonders als Dentalwerkstoffe, insbesondere als dentale Zemente, Füllungskomposite und Verblendmaterialien sowie als Materialien zur Herstellung von Prothesen, künstlichen Zähnen, Inlays, Onlays, Kronen und Brücken. Die Zusammensetzungen eignen sich primär zur intraoralen Anwendung durch den Zahnarzt zur Restauration geschädigter Zähne, d.h. zur therapeutischen Anwendung, z.B. als dentale Zemente, Füllungskomposite und Verblendmaterialien. Sie können aber auch nicht-therapeutisch (extraoral) eingesetzt werden, beispielsweise bei der Herstellung oder Reparatur von Dentalrestaurationen, wie Prothesen, künstlichen Zähnen, Inlays, Onlays, Kronen und Brücken.

Die erfindungsgemäßen Zusammensetzungen eignen sich außerdem zur Herstellung von Formkörpern für dentale aber auch für nicht dentale Zwecke, die z.B. mittels Gießen, Pressen und insbesondere durch generative Verfahren wie den 3D Druck hergestellt werden können.

Gegenstand der Erfindung ist auch die Verwendung einer Verbindung gemäß Formel I zur Herstellung eines radikalisch polymerisierbaren Werkstoffs, vorzugsweise eines medizintechnischen und insbesondere eines zahnmedizinischen Werkstoffs.

Die Erfindung wird im Folgenden anhand von Ausführungsbeispielen näher erläutert.

### Ausführungsbeispiele

### Beispiel 1:

### Synthese von 2,2',4,4',6,6'-hexaethyl- O'4,O4-(hexan-1,6-divl)bis(hepta-1,6-dien-2,4,4,6-tetracarboxylat) (1)

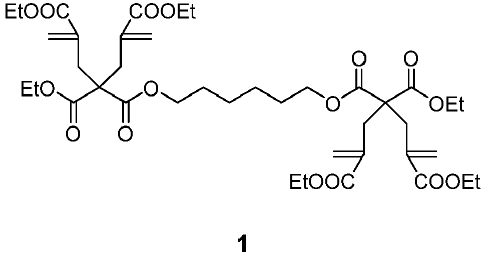

### 1. Stufe: Synthese von Diethyl-O,O'-(hexan-1,6-diyl)-dimalonat (ZP-1)

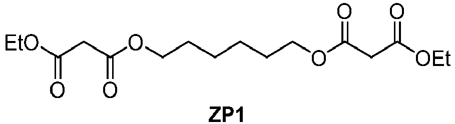

1,6-Hexandiol (42,3 mmol, 5,00 g) wurde in trockenem Dichlormethan (DCM, 150 ml) gelöst und die Apparatur mit Argon gespült. Die Lösung wurde in Eiswasser gekühlt, danach wurde zuerst Pyridin (105,8 mmol, 8,37 g) und dann Ethylmalonylchlorid (105,8 mmol, 15,93 g) hinzugefügt. Die Lösung wurde bei Raumtemperatur 4 h gerührt und anschließend mit 1N HCl (150 ml) gequencht. Die wässrige Phase wurde 3-mal mit DCM (150 ml) extrahiert, die vereinigten organischen Phasen wurden mit gesättigter NaHCO₃ Lösung (150 ml) gewaschen und über wasserfreiem Na₂SO₄ getrocknet. Das Lösungsmittel wurde abdestilliert, das gefärbte Rohprodukt über Kieselgel filtriert (Laufmittel PE:EE 4:1) und so das Reinprodukt **ZP1** als farbloses Öl in einer Ausbeute von 12,35 g (84% der Theorie) erhalten.

¹H-NMR: (400 MHz, CDCl₃) δ (ppm): 4,10 (8H, m, O-CH₂-), 3,29 (4H, s, C-CH₂-C), 1,59 (4H, m, CH₂), 1,32 (4H, m, CH₂), 1,28 (6H, m, -CH₃).

### 2. Stufe: Synthese von 2,2',4,4',6,6'-hexaethyl-O'4,O4-(hexan-1,6-diyl) bis (hepta-1, 6-dien-2,4,4,6-tetracarboxylat) (1)

Natriumhydrid (60 Gew.-%, Suspension in Mineralöl, 178,3 mmol, 4,28 g) wurde in einem Dreihalskolben vorgelegt und die Apparatur mit Argon gespült. Trockenes Tetrahydrofuran (THF) (100 ml) wurde hinzugefügt und die Reaktionslösung im Eisbad gekühlt. **ZP1** aus Stufe 1 (35,7 mmol, 12,35 g) wurde langsam zugetropft und die Reaktionslösung 2 h bei Raumtemperatur gerührt. Nach Zugabe von Ethylchloromethylacrylat (146,2 mmol, 21,72 g) wurde über Nacht gerührt und danach mit gesättigter NH₄Cl-Lösung (100 ml) gequencht. Die wässrige Phase wurde 3-mal mit Diethylether extrahiert (100 ml) und die vereinigten organischen Phasen über wasserfreiem Natriumsulfat getrocknet. Nach Abdampfen des Lösungsmittels wurde das Rohprodukt mittels Säulenchromatographie gereinigt (PE:EE 4:1) und das Reinprodukt als viskose Flüssigkeit in einer Ausbeute von 21,95 g (77% der Theorie) erhalten.

¹H-NMR: (400 MHz, CDCl₃) δ (ppm): 6,25 (4H, s, C=CH₂), 5,68 (4H, s, C=CH₂), 4,22-3,96 (16H, m, O-CH₂-), 2,95 (8H, s, C-CH₂-C), 1,57 (4H, m), 1,26 (22H, m). ¹³C-NMR: (100 MHz, CDCl₃) δ (ppm): 170,5 (C=O), 170,4 (C=O), 167,1 (C=O), 136,3 (C4), 128,7 (C2), 65,4 (C2), 61,5 (C2), 61,0 (C2), 57,6 (C4), 34,9 (C2), 28,4 (C2), 25,6 (C2), 14,3 (C1), 14,0 (C1).

### Beispiel 2 (Vergleichsbeispiel):

### Synthese von Diethyl-2,2'-(oxybis(methylen))diacrylat (2)

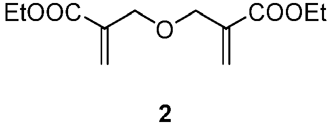

Die Synthese von Verbindung **2** wurde in Anlehnung an Tsuda et al., Polymer, 35 (1994), 3317-3328, durchgeführt:
Ethylacrylat (149,8 mmol, 15,0 g), Paraformaldehyd (149,8 mmol, 4,5 g) und 1,4-Di-azabicyclo(2.2.2)octan (1 g) wurden in einem Dreihalskolben vorgelegt und diese Lösung 3 Tage bei 95 °C gerührt. Nach Abkühlen der Lösung wurden 200 ml Petrolether hinzugefügt und die Lösung 3-mal mit 100 ml 3 %-iger HCl und 1-mal mit 100 ml Wasser gewaschen. Die organische Phase wurde über wasserfreiem Natriumsulfat getrocknet und das Lösungsmittel abgezogen. Das Rohprodukt wurde mittels Säulenchromatographie gereinigt und in einer Ausbeute von 11,7 g (65 % der Theorie) als farbloses Öl erhalten.

¹H-NMR: (400 MHz, CDCl₃) δ (ppm): 6,30 (2H, s, C=CH₂), 5,88 (2H, s, C=CH₂), 4,31-4,13 (8H, m, O-CH₂-), 1,29 (6H, t).

¹³C-NMR: (100 MHz, CDCl₃) δ (ppm): 165,9 (C=O), 137,4 (C4, 125,7 (C2), 69,0 (C2), 60,9 (C2), 14,3 (C1).

### Beispiel 3:

### 2,2', 4,4',6,6'-Hexaethyl-O'⁴, O⁴-{[(propan-2,2-diylbis(4,1-phenylen))bisoxy]bis-(propan-1, 2-diyl)}-bis(hepta-1,6-dien-2,4,4,6-tetracarboxylat), Isomerengemisch (3)

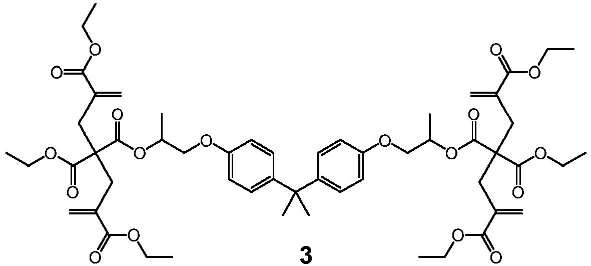

### 1. Stufe: O,O'-{[(Propan-2,2-diylbis-4,1-phenylen)-bisoxylbis(propan-1,2-diyl)}-dimalonsäurediethylester, Isomerengemisch (ZP-2)

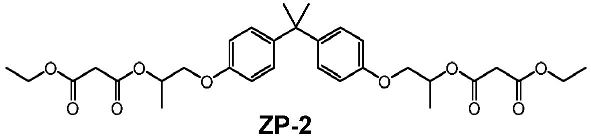

Zu einer Lösung von 1,1'-{[Propan-2,2-diylbis(4,1-phenylen)]bis(oxy)}bis(propan-2-ol) (Isomerengemisch, 17,22 g, 50,0 mmol) in DCM (100 ml) wurden Pyridin (9,89 g; 0,125 mol) und anschließend unter Eiskühlung Ethylmalonylchlorid (18,82 g; 0,125 mol) zugetropft und das Reaktionsgemisch wurde bei Umgebungstemperatur gerührt. Nach 4 h wurde Salzsäure (1*N*; 100 ml) zugegeben und die Phasen wurden getrennt. Die Wasserphase wurde mit DCM extrahiert (3x 50 ml). Die vereinigten organischen Phasen wurden mit gesättigter wässriger Natriumhydrogencarbonat-Lösung (100 ml) und gesättigter wässriger Natriumchlorid-Lösung (100 ml) gewaschen, über wasserfreiem Natriumsulfat getrocknet, filtriert und am Rotationsverdampfer eingeengt. Nach säulenchromatographischer Reinigung (SiO₂, *n-*Heptan/Ethylacetat 3:1) des Rohprodukts wurden 21,30 g (37,2 mmol; 74 % der Theorie) eines gelblichen Öls erhalten.

¹H-NMR (CDCl₃, 400 MHz): δ (Hauptisomer) = 7,12 (4H, m; Ar-H), 6,79 (4H, m; Ar-H), 5,29 (2H, m; O-CH), 4,18 (4H, m; O-CH₂), 3,98 (4H, m; O-CH₂), 3,36 (4H, s; CH₂), 1,62 (6H, s; CH₃), 1,37 (6H, d; CH-CH_{3;} J = 6,5 Hz), 1,25 (6H, t; CH₃; J = 7,1 Hz).

¹³C-NMR (CDCl₃, 100.6 MHz): δ (Hauptisomer) = 166,3 (C=O), 166,0 (C=O), 156,2 (Ar-C), 143,4 (Ar-C), 127,6 (Ar-CH), 113,8 (Ar-CH), 70,0 (O-CH₂), 69,6 (O-CH₂), 61,4 (O-CH), 41,6 (CH₂), 41,5 (C), 30,9 (CH₃), 16,4 (CH₃), 13,9 (CH₃).

### 2. Stufe: 2,2',4,4',6,6'-Hexaethyl O'⁴,O⁴-{[(propan-2,2-diylbis(4,1-phenylen))bis-oxy]bis-(propan-1,2-diyl)}-bis(hepta-1,6-dien-2,4,4,6-tetracarboxylat), Isomerengemisch (3)

Zu einer Suspension von Natriumhydrid (4,42 g, 0,184 mol) in THF (100 ml) wurde bei 0 °C eine Lösung von **ZP-2** (21,05 g, 36,8 mmol) in THF (50 ml) zugetropft und das Reaktionsgemisch wurde bei Umgebungstemperatur gerührt. Nach 3 h wurde unter Eiskühlung eine Lösung von 2-Chlormethylacrylsäureethylester (22,39 g, 0,151 mol) in THF (50 ml) zugetropft. Das Reaktionsgemisch wurde 24 h bei Umgebungstemperatur gerührt, dann wurde gesättigte wässrige Ammoniumchlorid-Lösung (100 ml) zugetropft. Wasser (100 ml) und Ethylacetat (250 ml) wurden zugegeben und die Phasen wurden getrennt. Die Wasserphase wurde mit Ethylacetat (2x 80 ml) extrahiert. Die vereinigten organischen Phasen wurden mit gesättigter wässriger Natriumchlorid-Lösung (2x 80 ml) gewaschen, über wasserfreiem Natriumsulfat getrocknet und filtriert. Das Filtrat wurde am Rotationsverdampfer eingeengt. Nach säulenchromatographischer Reinigung (SiO₂, n-Heptan/Ethylacetat 3:1) des Rohprodukts wurden 30,82 g (30,2 mmol; 82 % der Theorie) eines hochviskosen farblosen Öls erhalten.

¹H-NMR (CDCl₃, 400 MHz): δ (Hauptisomer) = 7,11 (4H, m; Ar-H), 6,77 (4H, m; Ar-H), 6,27 (4H, m; =CH), 5,74 (4H, m; =CH), 5,20 (2H, m; O-CH), 4,15 (8H, m; O-CH₂), 4,11 - 3,88 (8H, m; O-CH₂), 3,07 - 2,91 (8H, m; CH₂), 1,62 (6H, s; CH₃), 1,34 (6H, d; CH-CH_{3;} J = 6,5 Hz), 1,28 (6H, t; CH₃; J = 7,1 Hz), 1,18 (6H, t; CH₃; J = 7,1 Hz).

¹³C-NMR (CDCl₃, 100,6 MHz): δ (Hauptisomer) = 170,1 (C=O), 169,7 (C=O), 166,9 (C=O), 156,2 (Ar-C), 143,3 (Ar-C), 136,0 (=C), 135,8 (=C), 128,6 (=CH₂), 128,3 (=CH₂), 127,6 (Ar-CH), 113,7 (Ar-CH), 70,1 (O-CH), 69,3 (O-CH₂), 61,3 (O-CH₂), 60,8 (O-CH₂), 60,7 (O-CH₂), 57,4 (C), 41,5 (C), 34,3 (CH₂), 34,1 (CH₂), 30,9 (CH₃), 16,2 (CH₃), 14,0 (CH₂), 13,7 (CH₃).

### Beispiel 4:

### 2,2', 4,4',6,6'-Hexaethyl-O'⁴,O⁴-[(octahydro-1H-4,7-methanoinden-2,5-diyl)bis(methylen)]-bis(hepta-1,6-dien-2, 4,4, 6-tetracarboxylat), Isomerengemisch (4)

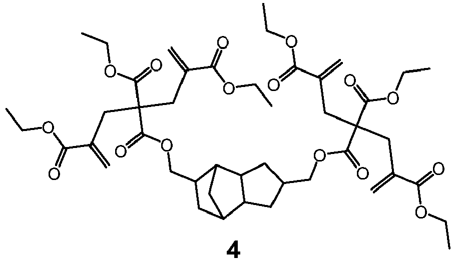

### 1. Stufe: O,O'-[(Octahydro-1H-4,7-methanoinden-2,5-diyl)bis(methylen)]-di-malonsäurediethylester, Isomerengemisch (ZP-3)

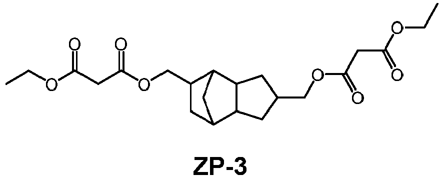

Zu einer Lösung von 4,8-Bis(hydroxymethyl)tricyclo[5.2.1.02,6]decan, Isomerengemisch (9,81 g, 50,0 mmol) in DCM (100 ml) wurden Pyridin (9,89 g; 0,125 mol) und anschließend unter Eiskühlung Ethylmalonylchlorid (18,82 g; 0,125 mol) zugetropft und das Reaktionsgemisch wurde bei Umgebungstemperatur gerührt. Nach 4 h wurde Salzsäure (1*N*; 100 ml) zugegeben und die Phasen wurden getrennt. Die Wasserphase wurde mit Dichlormethan extrahiert (3x 50 ml). Die vereinigten organischen Phasen wurden mit gesättigter wässriger Natriumhydrogencarbonat-Lösung (100 ml) und gesättigter wässriger Natriumchlorid-Lösung (100 ml) gewaschen, über wasserfreiem Natriumsulfat getrocknet, filtriert und am Rotationsverdampfer eingeengt. Nach säulenchromatographischer Reinigung (SiO₂, n-Heptan/Ethylacetat 1:1) des Rohprodukts wurden 20,75 g (48,9 mmol; 98 % der Theorie) eines gelblichen Öls erhalten.

¹H-NMR (CDCl₃, 400 MHz): δ = 4,25 - 4,16 (4H, m; O-CH₂), 4,04 - 3,87 (4H, m; O-CH₂), 3,40 - 3,34 (4H, m; CH₂), 2,56 - 1,32 (14H, m), 1,29 (6H, t; CH₃; *J* = 7,2 Hz).

¹³C-NMR (CDCl₃, 100,6 MHz): δ = 166,5 (C=O), 166,4 (C=O), 166,3 (C=O), 69,6 (O-CH₂), 69,0 (O-CH₂), 68,6 (O-CH₂), 68,6 (O-CH₂), 61,3 (O-CH₂), 49,1 (CH), 48,6 (CH), 45,3 (CH), 44,7 (CH), 44,6 (CH), 44,3 (CH), 43,5 (CH), 42,8 (CH), 42,5 (CH), 41,5

(CH₂), 41,3 (CH), 40,8 (CH), 40,5 (CH), 40,2 (CH₂), 40,0 (C), 39,2 (CH₂), 38,5 (CH), 37,9 (CH), 33,9 (CH), 33,8 (CH), 33,0 (CH), 32,3 (CH₂), 32,1 (CH₂), 30,5 (CH₂), 30,1 (CH₂), 27,9 (CH₂), 27,5 (CH₂), 25,0 (CH₂), 24,2 (CH₂), 13,9 (CH₃).

### 2. Stufe: 2,2,4,4',6,6'-Hexaethyl-O'⁴,O⁴-[(octahydro-1H-4,7-methanoinden-2,5-di-yl)bis(methylen)]-bis(hepta-1,6-dien-2,4,4,6-tetracarboxylat), Isomerengemisch (4)

Zu einer Suspension von Natriumhydrid (5,78 g, 0,241 mol) in Tetrahydrofuran (100 ml) wurde bei 0 °C eine Lösung von ZP-3, Isomerengemisch (20,45 g, 48,2 mmol) in THF (50 ml) zugetropft und das Reaktionsgemisch wurde bei Umgebungstemperatur gerührt. Nach 3 h wurde unter Eiskühlung eine Lösung von 2-Chlormethylacrylsäureethylester (29,35 g, 0,198 mol) in THF (50 ml) zugetropft. Das Reaktionsgemisch wurde 24 h bei Umgebungstemperatur gerührt, dann wurde gesättigte wässrige Ammoniumchlorid-Lösung (100 ml) zugetropft. Wasser (100 ml) und Ethylacetat (250 ml) wurden zugegeben und die Phasen wurden getrennt. Die Wasserphase wurde mit Ethylacetat (2x 80 ml) extrahiert. Die vereinigten organischen Phasen wurden mit gesättigter wässriger Natriumchlorid-Lösung (2x 80 ml) gewaschen, über wasserfreiem Natriumsulfat getrocknet und filtriert. Das Filtrat wurde am Rotationsverdampfer eingeengt. Nach säulenchromatographischer Reinigung (SiO₂, *n-*Heptan/Ethylacetat 3:1) des Rohprodukts wurden 30,30 g (34,7mmol; 72 % der Theorie) eines hochviskosen gelblichen Öls erhalten.

¹H-NMR (CDCl₃, 400 MHz): δ = 6,31 - 6,19 (4H, m; =CH), 5,74 - 5,61 (4H, m; =CH), 4,23 - 4,07 (12H, m; O-CH₂), 3,91 - 3,73 (4H, m; O-CH₂), 3,04 - 2,82 (8H, m; CH₂), 2,56 - 1,34 (14H, m), 1,33-1,21 (18H, m; CH₃).

¹³C-NMR (CDCl₃, 100,6 MHz): δ = 170,3 (C=O), 170,2 (C=O), 170,1 (C=O), 168,7 (C=O), 168,6 (C=O), 166,8 (C=O), 166,1 (C=O), 136,6 (=C), 136,0 (=C), 135,9 (=C), 128,4 (=CH₂), 127,5 (=CH₂), 69,1 (O-CH₂), 68,9 (O-CH₂), 68,6 (O-CH₂), 68,5 (O-CH₂), 61,3 (O-CH₂), 60,7 (O-CH₂), 57,3 (C), 50,8 (CH), 50,7 (CH), 48,8 (CH), 45,4 (CH), 44,7 (CH), 44,4 (CH), 43,5 (CH), 42,9 (CH), 42,8 (CH), 41,2 (CH), 40,9 (CH), 40,8 (CH), 40,2 (CH₂), 40,1 (CH), 39,2 (CH₂), 38,3 (CH), 38,0 (CH), 34,7 (CH₂), 34,6 (CH₂), 33,8 (CH), 33,6 (CH), 32,9 (CH), 32,4 (CH₂), 31,3 (CH₂), 30,5 (CH₂), 27,9 (CH₂), 27,4 (CH₂), 24,3 (CH₂), 14,0 (CH₃), 13,9 (CH₃), 13,8 (CH₃).

### Beispiel 5:

### Photopolymerisation und Bestimmung der Volumenkontraktion

Die Volumenkontraktion während der Polymerisation (Polymerisationsschrumpf) ΔV_{P} des erfindungsgemäßen Monomeren 1 aus Beispiel 1 (Beispiel 5, B1) wurde mittels Dichtemessung vor und nach der Polymerisation gemessen und mit dem kommerziell erhältlichen Referenzmonomer 1,10- Decandioldimethacrylat (D₃MA, CAS: 6701-13-9) (Beispiel 5: Vergleichsbeispiel V1) verglichen. D₃MA wurde als Referenz verwendet, weil es ebenfalls einen Alkylenspacer zwischen den polymerisationsfähigen Gruppen und denselben Atomabstand wie **1** besitzt. Zusätzlich wurde das bereits bekannte (vgl.: US 5,145,374) cyclopolymerisierbare Monomer **2** aus Beispiel 2 als Referenzmonomer verwendet (Beispiel 5: Vergleichsbeispiel V2). Die Dichte der Monomere wurde mittels 1 ml Pyknometer bestimmt, während die Dichte der ausgehärteten Polymere mittels Archimedes Methode bestimmt wurde. Die Monomere wurden mit 1 mol% BMDG (Bis(4-methoxybenzoyl)-diethylgermanium) als Photoinitiator versetzt, in eine Silikonform gegossen (15 × 10 × 4 mm) und mit einem Lichtofen (Modell Lumamat 100, Ivoclar AG, 400-500 nm, 20 mW cm⁻²) jeweils 10 min pro Seite gehärtet.

**Tabelle1: Polymerisationsschrumpf ΔV_{P} des erfindungsgemäßen Monomer 1 sowie der Vergleichsverbindungen D3MAa und Monomer 2**

| **Beispiel** | **Monomer** | **ρ_{Monomer} [q/mL]** | **PPolymer [g/mL]** | **Δ_{P}V [Vol-%]** |
|---|---|---|---|---|
| V1 | D₃MA | 0,962 | 1,091 | 12,8 |
| V2 | 2 | 1,055 | 1,220 | 12,2 |
| B1 | 1 | 1,127 | 1,194 | 4,4 |

Die Ergebnisse in Tabelle 1 belegen, dass das erfindungsgemäße Monomer **1** im Vergleich zu dem analogen Dimethacrylat-Monomer D₃MA und dem aus dem Stand der Technik bekannten cyclopolymerisierbaren Monomer **2** überraschenderweise einen deutlich geringen Polymerisationsschrumpf zeigt und somit einen klaren Vorteil bei möglichst formtreuen Applikationen darstellt.

### Beispiel 6:

### Reaktivitätsmessuna mittels RT-NIR-Photorheometrie

Zur Untersuchung der Photoreaktivität und vor allem der polymerisationsinduzierten Schrumpfungskraft des erfindungsgemäßen Monomeren 1 wurden die hergestellten Formulierungen B1, V1 und V2 aus Beispiel 5 mit einem Real-Time-Near-Infra-red (RT-NIR) Photorheometer MCR302 WESP von Anton Paar vermessen, das zur Umsatzkontrolle mit einem Bruker Vertex-80-IR-Spektrometer gekoppelt wurde. Es wurde ein PP-25-Messsystem verwendet, und der Messspalt wurde auf 0,2 mm eingestellt. Vor und während der Aushärtung (10 mW·cm⁻² auf Probenoberfläche; 400-500 nm; Omnicure 2000) wurden Speicher- und Verlustmodul der Proben im Oszillationsmodus (1 % Auslenkung, 1 Hz) gemessen. Gleichzeitig wurden während der Messung IR-Spektren der Probe mit einer Frequenz von ~ 4 Hz aufgezeichnet. Als Maß für die Photoreaktivität wurde das Erreichen des Gelpunkts (Schnittpunkt von Speicher- und Verlustmodul) und die Dauer bis zum Erreichen von 95 % des finalen Doppelbindungsumsatzes (t_{95%}) herangezogen. Zusätzlich wurden der Umsatz am Gelpunkt (DBC_{g}), der Gesamtumsatz (DBC) und die photopolymerisationsinduzierte Schrumpfungsspannung (Fₛ) ermittelt. Die erzielten Ergebnisse sind in Tabelle 2 zusammengefasst.

**Tabelle 2: Ergebnisse der Reaktivitätsmessungen mit Monomer 1 und den Referenz-Monomeren 2 und D₃MA**

| **Beispiel** | **Monomer** | **Gelpunkt [s]** | **DBC_{g} [%]** | **DBC [%]** | **t_{95%} [s]** | **Fₛ [N]** |
|---|---|---|---|---|---|---|
| V1 | D₃MA | 6,2 | 12 | 83 | 71 | -36 |
| V2 | 2 | 14,8 | 23 | 77 | 87 | -35 |
| B1 | 1 | 4,1 | 13 | 77 | 116 | -21 |

Betrachtet man den Gelpunkt der Monomere, erkennt man, dass das Monomer **1** diesen mit 4,1 s deutlich schneller erreicht als die Referenzverbindungen D₃MA und Monomer **2.** Dies belegt eine hohe Reaktivität von Monomer **1.** Der Umsatz am Gelpunkt hingegen ist mit 12-13% bei D₃MA und Monomer **1** ähnlich niedrig. Das cyclopolymerisierbare Referenzmonomer **2** zeigt einen deutlich höheren Umsatz am Gelpunkt (23 %), was durch die partielle Cyclopolymerisation und somit fehlende Quervernetzung zu erklären ist. Der finale Umsatz bewegt sich sowohl bei Monomer **1,** und den Vergleichsverbindungen **2** und D₃MA um ~80%, wobei D₃MA einen etwas höheren DBC erreicht. Dies ist auf eine höhere Flexibilität der langen Alkylenkette zurückzuführen, während Monomer **1** und auch teilweise Referenzverbindung **2** rigide Ringstrukturen bei der Polymerisation bilden und somit vor allem am Anfang der Polymerisation klar im Vorteil sind. Die auftretende Schrumpfkraft während der Polymerisation ist bei dem erfindungsgemäßen Monomer **1** mit -21 N über ein Drittel geringer als bei der Referenzsubstanz D₃MA mit -36 N. Dies zeigt den deutlichen Vorteil von Monomer **1** gegenüber dem konventionellen Dimethacrylat. Überraschenderweise zeigen die Ergebnisse, dass das ebenfalls cyclopolymerisierbare Vergleichsmonomer **2** ebenfalls eine sehr hohe Schrumpfkraft aufweist, was deutlich macht, dass die erfindungsgemäße Verbindung **1** beträchtliche Vorteile gegenüber der bekannten Vergleichsverbindung aufweist.

### Beispiel 7:

### Copolymerisation von Monomer 1 mit Dimethacrylate-Comonomeren

Um die Copolymerisationsfähigkeit des erfindungsgemäßen Monomeren 1 zu zeigen, wurde von einer Basis-Harzformulierung (äquimolares Gemisch der kommerziell erhältlichen Dimethacrylate Urethandimethacrylat (UDMA, Isomerengemisch; CAS: 72869-86-4) und 1,10-Decandioldimethacrylat (D₃MA) jeweils eine Komponente durch eine äquimolare Menge des Monomers **1** oder des Referenzmonomers **2** ersetzt und mittels RT-NIR-Photorheometrie die Reaktivität und auftretende Schrumpfkraft untersucht. Den Mischungen wurde jeweils **1** mol% BMDG (Bis(4-methoxybenzoyl)-diethylgermanium) als Photoinitiator zugefügt und die RT-NIR-Photorheometrie Messungen analog zu Beispiel 6 durchgeführt.

**Tabelle 3: Ergebnisse der Reaktivitätsmessungen der Copolymerisate**

| **Beispiel** | **Monomermischung** | **Gelpunkt [s]** | **DBC_{g} [%]** | **DBC [%]** | **t_{95%} [s]** | **Fₛ [N]** |
|---|---|---|---|---|---|---|
| V3 | D₃MA-UDMA | 1,5 | 16 | 78 | 79 | -32 |
| V4 | D₃MA-**2** | 9,3 | 12 | 84 | 94 | -42 |
| V5 | UDMA-**2** | 2,4 | 16 | 79 | 88 | -33 |
| B2 | D₃MA-**1** | 3,2 | 10 | 83 | 112 | -29 |
| B3 | UDMA-**1** | 3,0 | 18 | 75 | 82 | -23 |

Die Ergebnisse in Tabelle 3 belegen die gute Copolymerisation von Monomer **1** sowohl mit dem niedrigviskosen D₃MA, als auch mit dem zähflüssigen UDMA. Die Copolymerisation mit D₃MA führt zwar zu einem um 1,7 s späteren Gelpunkt, ist jedoch deutlich schneller als die Copolymerisation von Referenzmonomer **2** mit D₃MA (9,3 s). Die Gelierung der Copolymerisationen mit UDMA bewegt sich bei den Monomeren **1** und **2** im selben Rahmen, wobei Monomer **2** um 0,6 s schneller geliert. Sowohl der finale Umsatz (~80%) als auch **t_{95%}** (~90 s) liegen bei allen 5 Formulierungen im selben Bereich. Betrachtet man die auftretende Schrumpfkraft erkennt man erneut den großen Vorteil von Monomer **1.** Während die Referenzmischung V3 eine Schrumpfkraft von -32 N aufweist, führt die Substitution von D₃MA mit Monomer **1** zu einer Schrumpfkraft von nur -23 N. Verwendet man hingegen das Referenzmonomer **2,** kommt es bei Copolymerisation mit UDMA zu keiner Reduktion der Schrumpfkraft und bei der Copolymerisation mit D₃MA wird sogar ein Anstieg der Schrumpfkraft auf -42 N beobachtet. Somit konnte gezeigt werden, dass die Copolymerisation von Monomer **1** mit Dimethacrylaten kaum eine Verringerung der Reaktivität mit sich bringt, jedoch den Vorteil einer signifikanten Schrumpfkraftreduktion zeigt. Referenzmonomer **2** als cyclopolymerisierbares Monomer zeigte zwar ebenfalls eine hohe Reaktivität mit Dimethacrylaten, bewirkt jedoch eine Erhöhung der auftretenden Schrumpfkraft und ist gegenüber Monomer **1** damit klar im Nachteil.

## Patentansprüche

1. Dentalwerkstoff, der mindesten eine Verbindung der Formeln I enthält, 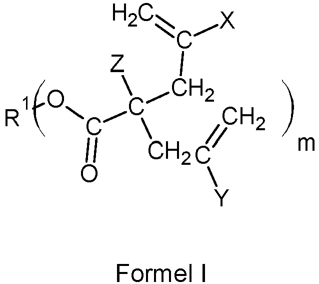 in der
R¹ ein m-wertiger, linearer, verzweigter oder cyclischer aliphatischer C₁-C₃₀-Kohlenwasserstoffrest oder ein aromatischer C₆-C₃₀-Kohlenwasserstoffrest ist, wobei der aliphatische oder aromatische Kohlenwasserstoffrest substituiert oder unsubstituiert sein kann und wobei aliphatische Kohlenwasserstoffreste durch eine oder mehrere Urethangruppen, Estergruppen, Sauerstoffatome und/oder Schwefelatome unterbrochen sein können,
X,Y,Z unabhängig voneinander jeweils -COOR², -CON(R³R⁴), ein aromatischer C₆-C₁₀-Kohlenwasserstoffrest oder -CN sind, wobei
R²,R³,R⁴ jeweils unabhängig voneinander Wasserstoff, ein linearer, verzweigter oder cyclischer aliphatischer C₁-C₃₀-Kohlenwasserstoffrest oder ein aromatischer C₆-C₃₀-Kohlenwasserstoffrest ist, wobei der aliphatische oder aromatische Kohlenwasserstoffrest substituiert oder unsubstituiert sein kann und wobei aliphatische Kohlenwasserstoffreste durch ein oder mehrere Sauerstoffatome unterbrochen sein können, und
m 2 oder 3 ist.

2. Dentalwerkstoff nach Anspruch 1, wobei die Variablen die folgenden Bedeutungen haben:
R¹ ein linearer, verzweigter oder cyclischer aliphatischer C₁-C₂₀-Kohlenwasserstoffrest, der durch eine oder mehrere, vorzugsweise 1 bis 3, Urethangruppen, Estergruppen und/oder Sauerstoffatome unterbrochen sein kann,
X,Y,Z unabhängig voneinander jeweils -COOR², ein aromatischer C₆-C₁₀-Kohlenwasserstoffrest oder -CN,
R² ein linearer, verzweigter oder cyclischer aliphatischer C₁-C₁₀-Kohlenwasserstoffrest, der durch ein oder mehrere, vorzugsweise 1 bis 3, Sauerstoffatome unterbrochen sein kann, und
m 2 oder 3.

3. Dentalwerkstoff nach Anspruch 1, wobei die Variablen die folgenden Bedeutungen haben:
R¹ ein linearer, verzweigter oder cyclischer, aliphatischer C₁-C₁₂-Kohlenwasserstoffrest, vorzugsweise ein gesättigter, verzweigter oder vorzugsweise linearer C₂- bis C₈-Kohlenwasserstoffrest, der durch ein oder mehrere, vorzugsweise 1 bis 3, Sauerstoffatome unterbrochen sein kann, und vorzugsweise nicht unterbrochen ist,
X,Y,Z unabhängig voneinander jeweils -COOR² oder Phenyl, vorzugsweise -COOR²,
R² ein linearer oder verzweigter C₁-C₄-Kohlenwasserstoffrest, vorzugsweise Ethyl, und
m 2 oder 3, vorzugsweise 2.

4. Dentalwerkstoff nach einem der Ansprüche 1 bis 3, der zusätzlich mindestens ein weiteres polymerisierbares Monomer, vorzugsweise mindestens ein radikalisch polymerisierbares mono- oder multifunktionelles (Meth)acrylat, besonders bevorzugt ein Methacrylat enthält.

5. Dentalwerkstoff nach Anspruch 4, der als weiteres Monomer ein oder mehrere multifunktionelle Monomere enthält, vorzugsweise Bisphenol-A-dimethacrylat, 2,2-Bis[4-(2-hydroxy-3-methacryloyloxypropyl)phenyl]propan (Bis-GMA), ethoxy- oder propoxyliertes Bisphenol-A-dimethacrylat, vorzugsweise 2-[4-(2-Methacryloyloxyethoxyethoxy)phenyl]-2-[4-(2-methacryloyloxyethoxy)phenyl]-propan) (SR-348c; enthält 3 Ethoxygruppen), 2,2-Bis[4-(2-methacryloyloxy-propoxy)phenyl]propan, 1,6-Bis-[2-methacryloyloxy-ethoxycarbonylamino]-2,2,4-trimethylhexan (UDMA), Di-, Tri- oder Tetraethylenglycoldimethacrylat, Trimethylolpropantrimethacrylat, Pentaerythrittetramethacrylat, Glycerindioder Glycerintrimethacrylat, 1,4-Butandioldimethacrylat, 1,10-Decandioldimethacrylat (D₃MA), 1,12-Dodecandioldimethacrylat, Bis(methacryloyl-oxymethyl)tricyclo-[5.2.1.0^{2,6}]decan (DCP) oder eine Mischung davon enthält.

6. Dentalwerkstoff nach einem der Ansprüche 4 bis 5, der als weiteres Monomer ein oder mehrere monofunktionelle Monomere enthält, vorzugsweise ein oder mehrere Monomethacrylate, besonders bevorzugt Benzyl-, Tetrahydrofurfuryl-, Isobornylmethacrylat, p-Cumyl-phenoxyethylenglycolmethacrylat (CMP-1E), 2-(2-Biphenyloxy)-ethylmethacrylat oder eine Mischung davon.

7. Dentalwerkstoff nach einem der Ansprüche 4 bis 6, der
0,5 bis 70 Gew.-%, vorzugsweise 1 bis 60 Gew.-% und besonders bevorzugt 3 bis 50 Gew.-% mindestens einer Verbindung der Formel I enthält, und/oder
insgesamt maximal 70 Gew.-%, vorzugsweise 1,5 bis 60 Gew.-% und besonders bevorzugt 2 bis 50 Gew.-% an multifunktionellen Comonomeren enthält, vorzugsweise Methacrylate, und/oder
insgesamt maximal 10 Gew.-%, vorzugsweise 0 bis 10 Gew.-% und besonders bevorzugt 0 bis 5 Gew.-% an monofunktionellen Comonomeren enthält, vorzugsweise Methacrylate,
jeweils bezogen auf die Gesamtmasse des Werkstoffs.

8. Dentalwerkstoff nach einem der Anspruch 4 bis 7, der als weiteres Monomer ein oder mehrere multifunktionelle Acrylate enthält, vorzugsweise Ethylenglycoldiacrylat, Hexandioldiacrylat, Tripropylenglycoldiacrylat, ethoxyliertes Bisphenol-A-diacrylat, Polyethylenglycol-200-diacrylat, Trimethylolpropantriacrylat, Pentaerythrittetraacrylat oder eine Mischungen davon.

9. Dentalwerkstoff nach einem der Ansprüche 1 bis 8, der zusätzlich mindestens einen Initiator für die radikalische Polymerisation durch UV-Licht, durch sichtbares Licht, einen thermischen Initiator und/oder einen Redoxinitiator enthält.

10. Dentalwerkstoff nach einem der Ansprüche 1 bis 9, der zusätzlich mindestens einen anorganischen Füllstoff enthält.

11. Dentalwerkstoff nach einem der Ansprüche 4 bis 10, der
a) 0,5 bis 70 Gew.-%, bevorzugt 1 bis 60 Gew.-% und besonders bevorzugt 3 bis 50 Gew.-% mindestens einer Verbindung der Formel I,
b) 0,01 bis 5 Gew.-%, bevorzugt 0,1 bis 3,0 Gew.-% und besonders bevorzugt 0,1 bis 1,0 Gew.-% mindestens eines Initiators, insbesondere eines Photoinitiators,
c) 1 bis 70 Gew.-%, bevorzugt 1,5 bis 60 Gew.-% und besonders bevorzugt 2 bis 50 Gew.-% mindestens eines radikalisch polymerisierbaren Comonomers,
d) 0 bis 85 Gew.-% mindestens eines Füllstoffs enthält,
jeweils bezogen auf die Gesamtmasse des Werkstoffs.

12. Dentalwerkstoff nach Anspruch 11, der 50 bis 85 Gew.-%, besonders bevorzugt 70 bis 80 Gew.-%, oder 10 bis 70 Gew.-%, besonders bevorzugt 60 bis 70 Gew.-% Füllstoff enthält.

13. Dentalwerkstoff nach einem der Ansprüche 1 bis 12 zur therapeutischen Verwendung als dentaler Zement, Füllungskomposit, Beschichtungs- oder Verblendmaterial.

14. Nicht therapeutische Verwendung eines Dentalwerkstoffs gemäß einem der Ansprüche 1 bis 12 zur Herstellung oder Reparatur von dentalen Prothesen, Inlays, Onlays, Kronen oder Brücken.

15. Verwendung einer Verbindung gemäß Formel I wie in Anspruch 1 definiert zur Herstellung eines medizintechnischen Werkstoffs.

## Claims

1. Dental material, which comprises at least one compound of formula I, 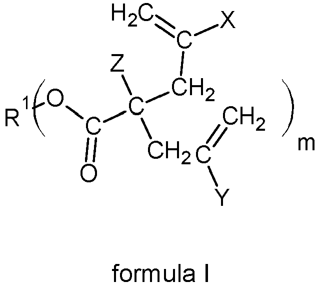 in which
R¹ is an m-valent, linear, branched or cyclic aliphatic C₁-C₃₀ hydrocarbon radical or an aromatic C₆-C₃₀ hydrocarbon radical, wherein the aliphatic or aromatic hydrocarbon radical can be substituted or unsubstituted and wherein aliphatic hydrocarbon radicals can be interrupted by one or more urethane groups, ester groups, oxygen atoms and/or sulfur atoms,
X,Y,Z independently of each other in each case are -COOR², -CON(R³R⁴), an aromatic C₆-C₁₀ hydrocarbon radical or -CN, wherein
R²,R³,R⁴ in each case independently of each other are hydrogen, a linear, branched or cyclic aliphatic C₁-C₃₀ hydrocarbon radical or an aromatic C₆-C₃₀ hydrocarbon radical, wherein the aliphatic or aromatic hydrocarbon radical can be substituted or unsubstituted and wherein aliphatic hydrocarbon radicals can be interrupted by one or more oxygen atoms, and
m is 2 or 3.

2. Dental material according to claim 1, wherein the variables have the following meanings:
R¹ a linear, branched or cyclic aliphatic C₁-C₂₀ hydrocarbon radical, which can be interrupted by one or more, preferably 1 to 3, urethane groups, ester groups and/or oxygen atoms,
X,Y,Z independently of each other in each case -COOR², an aromatic C₆-C₁₀ hydrocarbon radical or -CN,
R² a linear, branched or cyclic aliphatic C₁-C₁₀ hydrocarbon radical, which can be interrupted by one or more, preferably 1 to 3, oxygen atoms, and
m 2 or 3.

3. Dental material according to claim 1, wherein the variables have the following meanings:
R¹ a linear, branched or cyclic, aliphatic C₁-C₁₂ hydrocarbon radical, preferably a saturated, branched or preferably linear C₂ to C₈ hydrocarbon radical, which can be interrupted by one or more, preferably 1 to 3, oxygen atoms, and is preferably not interrupted,
X,Y,Z independently of each other in each case -COOR²or phenyl, preferably -COOR²,
R² a linear or branched C₁-C₄ hydrocarbon radical, preferably ethyl, and
m 2 or 3, preferably 2.

4. Dental material according to one of claims 1 to 3, which additionally comprises at least one further polymerizable monomer, preferably at least one radically polymerizable mono- or multifunctional (meth)acrylate, particularly preferably a methacrylate.

5. Dental material according to claim 4, which comprises one or more multifunctional monomers as further monomer, preferably bisphenol A dimethacrylate, 2,2-bis[4-(2-hydroxy-3-methacryloyloxypropyl)phenyl]propane (bis-GMA), ethoxylated or propoxylated bisphenol A dimethacrylate, preferably 2-[4-(2-methacryloyloxyethoxyethoxy)phenyl]-2-[4-(2-methacryloyloxyethoxy)phenyl]-propane) (SR-348c; contains 3 ethoxy groups), 2,2-bis[4-(2-methacryloyloxypropoxy)phenyl]propane, 1,6-bis-[2-methacryloyloxy-ethoxy-carbonylamino]-2,2,4-trimethylhexane (UDMA), di-, tri- or tetraethylene glycol dimethacrylate, trimethylolpropane trimethacrylate, pentaerythritol tetramethacrylate, glycerol di- or glycerol trimethacrylate, 1,4-butanediol dimethacrylate, 1,10-decanediol dimethacrylate (D₃MA), 1,12-dodecanediol dimethacrylate, bis(methacryloyloxymethyl)tricyclo-[5.2.1.0^{2,6}]decane (DCP) or a mixture thereof.

6. Dental material according to one of claims 4 to 5, which comprises one or more monofunctional monomers as further monomer, preferably one or more monomethacrylates, particularly preferably benzyl methacrylate, tetrahydrofurfuryl methacrylate, isobornyl methacrylate, p-cumyl-phenoxyethylene glycol methacrylate (CMP-1E), 2-(2-biphenyloxy)-ethyl methacrylate or a mixture thereof.

7. Dental material according to one of claims 4 to 6, which comprises
0.5 to 70 wt.-%, preferably 1 to 60 wt.-% and particularly preferably 3 to 50 wt.-% of at least one compound of formula I, and/or
in total a maximum of 70 wt.-%, preferably 1.5 to 60 wt.-% and particularly preferably 2 to 50 wt.-% multifunctional comonomers, preferably methacrylates, and/or
in total a maximum of 10 wt.-%, preferably 0 to 10 wt.-% and particularly preferably 0 to 5 wt.-% monofunctional comonomers, preferably methacrylates,
in each case relative to the total mass of the material.

8. Dental material according to one of claims 4 to 7, which comprises one or more multifunctional acrylates as further monomer, preferably ethylene glycol diacrylate, hexanediol diacrylate, tripropylene glycol diacrylate, ethoxylated bisphenol A diacrylate, polyethylene glycol 200 diacrylate, trimethylolpropane triacrylate, pentaerythritol tetraacrylate or a mixture thereof.

9. Dental material according to one of claims 1 to 8, which additionally comprises at least one initiator for the radical polymerization by UV light, by visible light, a thermal initiator and/or a redox initiator.

10. Dental material according to one of claims 1 to 9, which additionally comprises at least one inorganic filler.

11. Dental material according to one of claims 4 to 10, which comprises
a) 0.5 to 70 wt.-%, preferably 1 to 60 wt.-% and particularly preferably 3 to 50 wt.-% of at least one compound of formula I,
b) 0.01 to 5 wt.-%, preferably 0.1 to 3.0 wt.-% and particularly preferably 0.1 to 1.0 wt.-% of at least one initiator, in particular of a photoinitiator,
c) 1 to 70 wt.-%, preferably 1.5 to 60 wt.-% and particularly preferably 2 to 50 wt.-% of at least one radically polymerizable comonomer,
d) 0 to 85 wt.-% of at least one filler,
in each case relative to the total mass of the material.

12. Dental material according to claim 11, which comprises 50 to 85 wt.-%, particularly preferably 70 to 80 wt.-%, or 10 to 70 wt.-%, particularly preferably 60 to 70 wt.-% filler.

13. Dental material according to one of claims 1 to 12 for therapeutic use as dental cement, filling composite, coating or veneering material.

14. Non-therapeutic use of a dental material according to one of claims 1 to 12 for the production or repair of dental prostheses, inlays, onlays, crowns or bridges.

15. Use of a compound according to formula I, as defined in claim 1, for the preparation of a medical material.

## Revendications

1. Matériau dentaire qui contient au moins un composé de formule I : 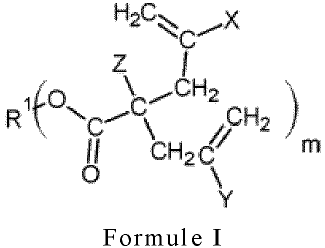 dans laquelle
- R¹ représente un reste m-valent d'hydrocarbure aliphatique linéaire, ramifié ou cyclique en C₁-C₃₀ ou un reste d'hydrocarbure aromatique en C₆-C₃₀, lequel reste d'hydrocarbure aliphatique ou aromatique peut être porteur de substituant(s) ou non, étant entendu que le reste d'hydrocarbure aliphatique peut être interrompu par un ou plusieurs groupe(s) uréthane, groupe(s) ester, atome(s) d'oxygène et/ou atome(s) de soufre,
- X, Y et Z représentent chacun, indépendamment les uns des autres, un groupe de formule -COOR² ou -CON(R³R⁴), un reste d'hydrocarbure aromatique en C₆-C₁₀, ou un groupe cyano,
étant entendu que R², R³ et R⁴ représentent chacun, indépendamment les uns des autres, un atome d'hydrogène, un reste d'hydrocarbure aliphatique linéaire, ramifié ou cyclique en C₁-C₃₀ ou un reste d'hydrocarbure aromatique en C₆-C₃₀, lequel reste d'hydrocarbure aliphatique ou aromatique peut être porteur de substituant(s) ou non, étant entendu que le reste d'hydrocarbure aliphatique peut être interrompu par un ou plusieurs atome(s) d'oxygène,
- et l'indice m vaut 2 ou 3.

2. Matériau dentaire conforme à la revendication 1, dans lequel les symboles ont les significations suivantes :
- R¹ représente un reste d'hydrocarbure aliphatique linéaire, ramifié ou cyclique en C₁-C₂₀, qui peut être interrompu par un ou plusieurs, de préférence de 1 à 3, groupe(s) uréthane, groupe(s) ester et/ou atome(s) d'oxygène,
- X, Y et Z représentent chacun, indépendamment les uns des autres, un groupe de formule -COOR², un reste d'hydrocarbure aromatique en C₆-C₁₀, ou un groupe cyano,
étant entendu que R² représente un reste d'hydrocarbure aliphatique linéaire, ramifié ou cyclique en C₁-C₁₀, qui peut être interrompu par un ou plusieurs, de préférence de 1 à 3, atome(s) d'oxygène,
- et l'indice m vaut 2 ou 3.

3. Matériau dentaire conforme à la revendication 1, dans lequel les symboles ont les significations suivantes :
- R¹ représente un reste d'hydrocarbure aliphatique linéaire, ramifié ou cyclique en C₁-C₁₂, de préférence un reste d'hydrocarbure saturé, ramifié ou de préférence linéaire en C₂-C₈, qui peut être interrompu par un ou plusieurs, de préférence de 1 à 3, atome(s) d'oxygène, mais qui de préférence n'est pas interrompu,
- X, Y et Z représentent chacun, indépendamment les uns des autres, un groupe de formule -COOR² ou un groupe phényle, et de préférence un groupe de formule -COOR²,
étant entendu que R² représente un reste d'hydrocarbure aliphatique linéaire ou ramifié en C₁-C₄, de préférence un groupe éthyle,
- et l'indice m vaut 2 ou 3, de préférence 2.

4. Matériau dentaire conforme à l'une des revendications 1 à 3, qui contient en outre au moins un autre monomère polymérisable, de préférence au moins un acrylate ou méthacrylate monofonctionnel ou polyfonctionnel, polymérisable par voie radicalaire, et en particulier, un méthacrylate.

5. Matériau dentaire conforme à la revendication 4, qui contient, en tant qu'autre monomère, un ou plusieurs monomère(s) polyfonctionnel(s), et de préférence, du diméthacrylate de bisphénol A, du 2,2-bis[4-(2-hydroxy-3-méthacryloyloxy-propyl)-phényl]-propane (bis-GMA), du diméthacrylate de bisphénol A éthoxylé ou propoxylé, de préférence du 2-[4-(2-méthacryloyloxy-éthoxy-éthoxy)-phényl]-2-[4-(2-méthacryloyloxy-éthoxy)-phényl]-propane (SR-348c, comportant 3 groupes éthoxy), du 2,2-bis[4-(2-méthacryloyloxy-propoxy)-phényl]-propane, du 1,6-bis(2-méthacryloyloxy-éthoxy-carbonyl-amino)-2,2,4-triméthyl-hexane (UDMA), du diméthacrylate de diéthylèneglycol, de triéthylèneglycol ou de tétraéthylèneglycol, du triméthacrylate de triméthylolpropane, du tétraméthacrylate de pentaérythritol, du diméthacrylate ou triméthacrylate de glycérol, du diméthacrylate de butane-1,4-diol, du diméthacrylate de décane-1,10-diol (D₃MA), du diméthacrylate de dodécane-1,12-diol, du bis(méthacryloyloxy-méthyl)-tricyclo[5.2.1.0^{2,6}]décane (DCP), ou un mélange de ces composés.

6. Matériau dentaire conforme à l'une des revendications 4 et 5, qui contient, en tant qu'autre monomère, un ou plusieurs monomères monofonctionnels, de préférence un ou plusieurs monométhacrylates, et en particulier, du méthacrylate de benzyle, de tétrahydrofuryle ou d'isobornyle, du méthacrylate de p-cumyl-phénoxy-éthylèneglycol (CMP-1E), du méthacrylate de 2-(biphén-2-yl-oxy)-éthyle, ou un mélange de ces composés.

7. Matériau dentaire conforme à l'une des revendications 4 à 6, qui
- contient de 0,5 à 70 % en poids, de préférence de 1 à 60 % en poids, et en particulier de 3 à 50 % en poids d'au moins un composé de formule I,
- et/ou contient, au total, au maximum 70 % en poids, de préférence de 1,5 à 60 % en poids, et en particulier de 2 à 50 % en poids de comonomères polyfonctionnels, de préférence méthacrylates,
- et/ou contient, au total, au maximum 10 % en poids, de préférence de 0 à 10 % en poids, et en particulier de 0 à 5 % en poids de comonomères monofonctionnels, de préférence méthacrylates,
chacun de ces pourcentages étant rapporté au poids total du matériau.

8. Matériau dentaire conforme à l'une des revendications 4 à 7, qui contient, en tant qu'autre monomère, un ou plusieurs acrylates polyfonctionnels, de préférence du diacrylate d'éthylèneglycol, du diacrylate d'hexanediol, du diacrylate de tripropylèneglycol, du diacrylate de bisphénol A éthoxylé, du diacrylate de polyéthylène-glycol-200, du triacrylate de triméthylolpropane, du tétraacrylate de pentaérythritol, ou un mélange de ces composés.

9. Matériau dentaire conforme à l'une des revendications 1 à 8, qui contient en outre au moins un amorceur de polymérisation par voie radicalaire agissant sous lumière ultraviolette ou visible, un amorceur thermique et/ou un amorceur redox.

10. Matériau dentaire conforme à l'une des revendications 1 à 9, qui contient en outre au moins une charge inorganique.

11. Matériau dentaire conforme à l'une des revendications 4 à 10, qui contient
a) de 0,5 à 70 % en poids, de préférence de 1 à 60 % en poids, et en particulier de 3 à 50 % en poids d'au moins un composé de formule I,
b) de 0,01 à 5 % en poids, de préférence de 0,1 à 3,0 % en poids, et en particulier de 0,1 à 1,0 % en poids d'au moins un amorceur, en particulier d'un photoamorceur,
c) de 1 à 70 % en poids, de préférence de 1,5 à 60 % en poids, et en particulier de 2 à 50 % en poids d'au moins un comonomère polymérisable par voie radicalaire,
d) et de 0 à 85 % en poids d'au moins une charge,
chacun de ces pourcentages étant rapporté au poids total du matériau.

12. Matériau dentaire conforme à la revendication 11, qui contient de 50 à 85 % en poids et en particulier de 70 à 80 % en poids, ou bien de 10 à 70 % en poids et en particulier de 60 à 70 % en poids, de charge.

13. Matériau dentaire, conforme à l'une des revendications 1 à 12, pour utilisation thérapeutique en tant que ciment dentaire, matériau composite d'obturation, ou matériau de revêtement ou de placage.

14. Utilisation non thérapeutique d'un matériau dentaire, conforme à l'une des revendications 1 à 12, pour la fabrication ou la réparation de prothèses dentaires, d'inlays, d'onlays, de couronnes ou de bridges.

15. Utilisation d'un composé de formule I, tel que défini dans la revendication 1, pour la fabrication d'un matériau technico-médical.
